# EUROPÄISCHE PATENTSCHRIFT

(11) **EP 0 446 797 B1**
(45) Veröffentlichungstag und Bekanntmachung des Hinweises auf die Patenterteilung: **07.06.2000**
(21) Anmeldenummer: 91103555.8
(22) Anmeldetag: 08.03.1991
(51) Int. Cl.: C07K 14/00, C07K 17/06, C07K 7/06, C12N 15/52, G01N 33/543, G01N 33/68

(54) **Synthetische Peptide, die Sequenzen aus Faktor VIIa enthalten und deren Verwendung**
Synthetic peptides containing factor VIIa sequences and their use
Peptides synthétiques comportant des séquences du facteur VIIa et leur utilisation

(30) Priorität: 13.03.1990 DE 4007902
(43) Veröffentlichungstag der Anmeldung: 18.09.1991
(73) Patentinhaber: Dade Behring Marburg GmbH, 35001 Marburg (DE)
(72) Erfinder: Pelzer, Hermann, Dr., W-3550 Marburg (DE); Stüber, Werner, Dr., W-3551 Lahntal (DE)

(56) Entgegenhaltungen:
- EP-A- 0 355 572
- WO-A-90/03390
- PROCEEDINGS OF THE NATIONAL ACADEMY OF SCIENCES OF USA Bd. 83, Nr. 8, April 1986, WASHINGTON US Seiten 2412 - 2416 HAGEN, F. ET AL. 'Characterization of a cDNA coding for human factor VII'
- JOURNAL OF CLINICAL PATHOLOGY Bd. 41, Nr. 3, M{rz 1988, LONDON, GB Seiten 337 - 341 TAKASE, T. ET AL. 'Monoclonal antibodies to human factor VII: a one step immunoradiometric assay for VII:Ag'
- Proc Natl Acad Sci USA 84 (1987) 5158-62
- J Biol Chem 263 (1988) 14868
- Biochemistry 27 (1988) 7785

## Beschreibung

Die Erfindung bezieht sich auf synthetische Peptide, die bestimmte Teilsequenzen aus Faktor VIIa enthalten, ihre Synthese und die Verwendung dieser Peptide zum Immunisieren eines Tieres und zur Reinigung von spezifischen Antikörpern gegen die genannten Peptide, auf Antikörper gegen diese Peptide und die Verwendung dieser Antikörper und Peptide in Therapie und Diagnostik.

Der Organismus ist durch das Gerinnungssystem vor Blutverlust geschützt. Es kommt dabei innerhalb der Gerinnungskaskade zur Bildung eines Faktor VII/Tissue Factor (TF)-Komplexes, welcher durch limitierte Proteolyse, wahrscheinlich durch Spuren von Faktor Xa, zum Faktor VIIa/TF-Komplex umgewandelt wird. Der Faktor VIIa/TF-Komplex besitzt eine gegenüber freiem Faktor VIIa vielfach erhöhte proteolytische Aktivität. Die spezifische Spaltung des Faktor VII-Moleküls erfolgt an der dem Tetrapeptid Pro-Gln-Gly-Arg folgenden Peptidbindung. Durch diese Spaltung entsteht ein zweikettiges Faktor VIIa-Molekül, bestehend aus einer leichten Kette mit 152 Aminosäuren sowie einer schweren Kette mit 254 Aminosäuren. Beide Ketten werden durch eine Disulfidbindung zusammengehalten. Bei Aktivierung, d. h. Spaltung, des einkettigen Faktor VII-Moleküls zum zweikettigen Faktor VIIa-Molekül entsteht auf der leichten bzw. schweren Kette des Faktor VIIa-Moleküls eine neue carboxy- bzw. aminoterminale Aminosäuresequenz. Mit Hilfe eines spezifischen Antikörpers, der ausschließlich die nach Entstehung von Faktor VIIa neugebildete carboxy- bzw. aminoterminale Aminosäuresequenz erkennt, nicht jedoch den nativen Faktor VII, läßt sich eine spezifische Bestimmung von Faktor VIIa im Blut oder Plasma durchführen. Dadurch wird eine Quantifizierung der in der Initialphase der exogenen Gerinnung entstehenden Gerinnungskapazität möglich.

Die Bestimmung der funktionellen Aktivität von Faktor VII unter Verwendung von Faktor VII-Mangelplasma ist bekannt John T. Brandt et al., Am.J. Clin. Pathol. 85 (1986), 583 - 589). Hierbei bestimmt man den gerinnungszeitverkürzenden Effekt von verdünntem Plasma in einem System, welches alle zum Gerinnungsvorgang notwendigen Faktoren mit Ausnahme von Faktor VII enthält. Der Test erfaßt vollständig den in der Plasmaprobe aktivierbaren Anteil von Faktor VII, vermag jedoch nicht die Konzentration von bereits vorhandenem aktivem Faktor VIIa zu erfassen.

Es ist auch ein Verfahren zur Quantifizierung (C. Bayer et al., Thrombosis and Haemostasis, 56 (3) (1986), 250 - 255) von Faktor VII unter Verwendung von Radio- bzw. Enzymimmunoassays bekannt. Die hierfür notwendigen Antikörper werden erzeugt, indem aus Plasma gereinigter Faktor VII zum Immunisieren von Tieren verwendet wird. Die erhaltenen Antikörper eignen sich zur quantitativen Bestimmung von Faktor VII, ermöglichen aber keine Unterscheidung zwischen inaktivem Faktor VII und aktivem Faktor VIIa.

Die cDNA, die für den humanen Faktor VII kodiert, wurde sequenziert (HAGEN et alt. (1986), Proc. Natl. Acad. Sci, USA 83 pp. 2412 - 2416). Ein Bestimmungsverfahren für Vaktor VII wurde schon beschrieben (TAKASE et al. (1988), J.Clin. Pathol. 41, pp. 337 - 341). Die dabei verwendeten Antikörper erkennen jedoch lediglich den intakten Faktor VII. Die Erkennungssequenz für Faktor Xa wurde von O'Hara et al. beschrieben (Proc. Natl. Acad. Sci. USA (1987) 84 pp. 5158-5162). TAKEYA et al. haben die Reinigung und die vollständige Aminosäuresequenz des des bovinen Faktor VII sowie von Fragmenten davon beschrieben. THIM et al. erwähnen unter anderem Peptidsequenzen aus dem Bereich der Spaltstelle des humanen Faktor VIIa.

Der vorliegenden Erfindung lag daher die Aufgabe zugrunde, spezifische Antikörper gegen Faktor VIIa zur Verfügung zu stellen. Ferner lag die Aufgabe zugrunde, ein Testverfahren unter Verwendung spezifischer Faktor VIIa-Antikörper zu entwickeln, welches eine sensitive und exakte Quantifizierung von Faktor VIIa bzw. Faktor VIIa/TF-Komplex in biologischen Flüssigkeiten ermöglicht.

Diese Aufgabe wird erfindungsgemäß durch die Verwendung bestimmter synthetische Peptide gelöst, die Aminosäuresequenzen aufweisen, die teilweise der Aminosäuresequenz von Faktor VIIa entsprechen und antigen sind.

Gegenstand der Erfindung ist ein diagnostisches in vitro Verfahren zur Bestimmung von F VIIa bzw. F VIIa/TF-Komplex, wobei Antikörper eingesetzt werden, die durch Immunisierung mit Peptiden aus F VIIa erhältlich sind, wobei diese Peptide mindestens die 4 carboxy-terminalen Aminosäuren -Pro-Gln-Gly-Arg-OH und/oder aminoterminalen H-Ile-Val-Gly-Gly endständig enthalten.

Zur Immunisierung werden Dekapeptide eingesetzt, die gegebenenfalls ein N- oder C-terminal eingefügtes Cystein enthalten.

Vorteilhaft sind die Dekapeptide Cys-Arg-Asn-Ala-Ser-Lys-Pro-Gln-Gly-Arg und Ile-Val-Gly-Gly-Lys-Val-Cys-Pro-Lys-Gly. Zur besseren Immunisierung sind diese Dekapeptide an ein Trägermolekül gekoppelt.

Außerdem ist Gegenstand der Erfindung ein solches diagnostisches Verfahren, bei dem ein Antikörper an eine Festphase gebunden ist und der zweite Antikörper eine nachweisbare Funktion trägt.

Zusätzlich ist Gegenstand der Erfindung ein diagnostisches Verfahren , bei dem der erste Antikörper, erhältlich durch Immunisierung mit Peptiden aus F VIIa, wobei diese Peptide mindestens die 4 carboxy-terminalen Aminosäuren -Pro-Gln-Gly-Arg-OH und/oder aminoterminalen H-Ile-Val-Gly-Gly endständig enthalten, an die Festphase gebunden ist und der zweite Antikörper, der eine Nachweisfunktion trägt, gegen TF gerichtet ist.

Ein weiterer Gegenstand der Erfindung sind Konjugate aus synthetischen Peptiden aus F VIIa, die mindestens die 4 carboxyterminalen Aminosäuren (-Pro-Gly-Arg-OH) und/oder aminotermi-nalen (H-Ile-Val-Gly-Gly-) Aminosäuren endständig ent- halten, wobei gegebenenfalls ein Cystein auf dem den 4 genannten terminalen Aminosäuren entgegengesetzten Ende angefügt ist, ausgenommen das Undekapeppeptid Glu-Lys-Arg-Asn-Ala-Ser-Lys-Pro-Gly-Pro-Arg und die jeweilige vollständige Kette von F VIIa und einem Trägermolekül.

Zur besseren Immunisierung sind diese Moleküle an ein Trägermolekül gekoppelt.

Die erfindungsgemäß verwendeten Peptide können nach dem Fachmann an sich bekannten Verfahren hergestellt werden, z. B. können dabei geschützte Aminosäurederivate oder Peptidsegmente in Lösung oder an einer Festphase aneinander gekoppelt werden und durch Abspalten der Schutzgruppen sowie im Falle einer Festphase durch Abspaltung vom Trägerharz erfindungsgemäße Peptid erhalten werden. Als temporäre Schutzgruppe werden dabei bevorzugterweise die Fmoc-Gruppe, die permanenten Schutzgruppen für die Seitenfunktionen auf t-Butyl/Boc-Basis, für Arg die Pmc- oder Mtr-Gruppe und für Cys die tert.-Butylmercaptogruppen oder Tritylgruppen benutzt. Die Immobilisierung der C-terminalen Aminosäure erfolgt über p-Alkoxybenzylester gruppen, die an einen üblicherweise für die Peptidsynthese geeigneten polymeren Träger, vorzugsweise quervernetztem Polystyrol, gebunden sind. Der Peptidaufbau erfolgt unter repetitiver Fmoc-Abspaltung, vorzugsweise mit 20 % Piperidin in DMF (Dimethylformamid) (V/V) und Kopplung der folgenden, geschützten Aminosäure, vorzugsweise mit einem Carbodiimid in Gegenwart von HOBT. Das Aminosäurederivat wird hierzu in einem Überschuß, vor zugsweise 3fach, während 1 - 1.5 Stunden in DMF gekoppelt. Nach jedem Arbeitsschritt, Fmoc-Abspaltung bzw. Kondensationsschritt, wird das Harz mit kleinen (15 ml/g) Portionen DMF bzw. Isopropanol je 3mal gewaschen. Die Abspaltung der erfindungsgemäßen Peptide erfolgt acidolytisch unter gleichzeitiger Freisetzung der Seitenkettenfunktionen. Gegebenenfalls freizulegende Sulfhydrylgruppen werden mit Tri-n-Butylphosphin in einem Alkohol, beispielsweise Trifluorethanol oder mit DTT in Wasser "entschützt". Im Falle der Cys (Trt)-Entschützung ist ein separater Arbeitsschritt bei Benutzung von Ethandithiol als Scavenger unnötig. Die Reinigung der Peptide kann z. B. erfolgen über Ionenaustauschchromatographie, reversed-phase Chromatography und Gelpermeationschromatographie. Die korrekte Zusammensetzung der Peptide sowie die Peptidgehalte werden durch Aminosäureanalyse bestimmt.

Die Verwendung von synthetischen Peptiden als Antigene bei der Immunisierung von Tieren führt zur Erzeugung von speziell gegen das in diesem Peptid exponierte Hapten gerichteten Antikörpern. Die so erzeugten Antikörper sind daher spezifisch für jeweils eine einzige Antikörperbindungsstelle des gesamten Proteins, aus dem die Peptidsequenz abgeleitet worden ist. Die Verwendung von synthetischen Peptiden hat gegenüber der Verwendung von herkömmlich gereinigtem Faktor VIIa entscheidende Vorteile; synthetische Peptide lassen sich in großem Maßstab und in hoher Reinheit herstellen, so daß ein aufwendiges Isolieren und Reinigen von natürlichem Faktor VIIa entfällt. Während die Reinigung synthetischer Peptide von Synthesenebenprodukten gut etabliert ist, führen auch technisch aufwendige Anreicherungs- und Reinigungsverfahren für natürlichen Faktor VIIa stets zu Präparationen, die einen sehr geringen, jedoch als Antigen wirksamen Anteil unerwünschter Peptide, beispielsweise nativen Faktor VII, enthalten. Darüber hinaus erhält man bei Verwendung von komplettem Faktor VII bzw. Faktor VIIa als Immunisierungsantigen stets eine Vielzahl von Antikörperpopulationen, die letztlich gegen sämtliche in diesen Proteinen exponierten Haptene gerichtet sind; diese Vielfalt unterschiedlicher Antikörper erschwert das Auffinden spezifischer, ausschließlich gegen Faktor VIIa gerichteter Antikörper.

Es hat sich nun gezeigt, daß Antikörper, die gegen ein Peptid oder Polypeptid gerichtet sind, das an seinem C- bzw. N-terminalen Ende die Erkennungssequenz für Faktor Xa enthält, ausschließlich spezifisch mit Faktor VIIa reagieren, nicht jedoch mit dem intakten, ungespaltenen Faktor VII.
Die durch Einwirkung von Faktor Xa auf Faktor VII entstandenen 2 Ketten, die C- bzw. N-terminal die Faktor Xa-Erkennungssequenz tragen, sind 152 bzw. 254 Aminosäuren lang. Zur Immunisierung eignen sich sowohl die kompletten Polypeptide als auch Teilsequenzen dieser Peptide, die aber nach wie vor am C- bzw. N-Terminus die Faktor Xa-Erkennungssequenz aufweisen müssen. Eine besonders bevorzugte Ausführungsform sieht die Verwendung von Dekapeptiden vor, beispielsweise mit den Sequenzen Cys-Arg-Asn-Ala-Ser-Lys-Pro-Gln-Gly-Arg bzw. Ile-Val-Gly-Gly-Lys-Val-Cys-Pro-Lys-Gly.

Für die genannten Fälle ist wichtig, daß die carboxy- bzw. aminoterminale Sequenz des Moleküls exponiert ist und zur Immunisierung führt.

In Anbetracht der für die Peptide vorgesehenen Verwendung ist es sinnvoll, Aminosäuren mit reaktiven Seitengruppen so in die Peptide einzuführen, daß sie die Struktur des Haptens nicht beeinflussen. Zweckmäßigerweise wird aus diesem Grund N- bzw. C-terminal gegebenenfalls Cystein angefügt, dessen freie SH-Gruppe zur Kopplung via Thioether an viele Träger geeignet ist. Bevorzugt wird z. B. das durch das oben genannte Peptid repräsentierte Antigen in Form des Dekapeptides Cys-Arg-Asn-Ala-Ser-Lys-Pro-Gln-Gly-Arg zur Verfügung gestellt.

Die Herstellung des zur Immunisierung eingesetzten Peptides kann sowohl auf dem Fachmann an sich bekannte Art durch chemische Synthese geschehen, als auch durch Reinigung eines gentechnologisch bereitgestellten Polypeptides.

Peptide, die zur Immunisierung eingesetzt werden sollen oder solche, die Verwendung als Immunoadsorbens finden sollen, werden sinnvollerweise an ein Trägermolekül gekoppelt. Kopplungsverfahren sind dem Fachmann an sich bekannt und in der Literatur beschrieben (Nakane, P.K. et al., J. Histochem. Cytochem. 22 (1974), 1084 - 1091). Trägermoleküle im Sinne dieser Erfindung können sein natürliche oder synthetische Makromoleküle wie sie der Fachmann zur Erzeugung eines immunreaktiven Konjugats verwendet, wie z. B. Albumin, Ovalbumin, Keyhole Limpet Hemocyanin oder Polysaccharide. In einer bevorzugten Ausführungsform wird das Peptid oder Polypeptid an das Hemocyanin einer marinen Napfschnecke, das Keyhole Limpet Hemocyanin, gebunden.

Bei Verwendung der erfindungsgemäßen synthetischen Peptide als Immunadsorbens empfiehlt sich die Kopplung an Materialien, die sich zum Bereitstellen fester Matrizes eignen. Trägermoleküle in diesem Sinne sind unlösliche Polymere, wie sie der Fachmann zur Immobilisierung von Proteinen und Peptiden verwendet, wie z. B. Polystyrol, Nylon, Agarose oder magnetisierbare Partikel. Die feste Phase kann dabei in beliebiger Form z. B. als Röhrchen, Vlies, Kugel oder Mikropartikel vorliegen.

Eine bevorzugte Ausführungsform sieht die Kopplung von Peptiden, z. B. der oben erwähnten Dekapeptide, an bromcyanaktivierte Sepharose vor.

Die Immunisierung geeigneter Tiere mit trägergebundenen Peptiden führt reproduzierbar zur Bildung von Antikörpern. Eine bevorzugte Tierspezies für Immunisierung und Antikörpergewinnung ist hierbei das Kaninchen; darüber hinaus können auch Mäuse zur Immunisierung verwendet werden.

Aus einem solchen, erfindungsgemäß mit synthetischen Peptiden im Tier erzeugten Antiserum kann die für spezifische Tests relevante Immunglobulinfraktion durch übliche immunadsorptive Methoden angereichert werden. Bevorzugt ist es in diesem Falle jedoch, als Material für eine solche zur Immunadsorption eingesetzte Matrix ebenfalls ein an einen Träger gekoppeltes Peptid zu verwenden, das die gleiche antigene Determinante wie das zur Immunisierung eingesetzte Peptid aufweist. Das zur immunadorptiven Reinigung verwendete Peptid kann auch eine verkürzte Aminosäurensequenz aufweisen; Voraussetzung zur Verwendung in der immunadsorptiven Reinigung des gewünschten Antikörpers ist lediglich, daß die von diesem kürzeren Polypeptid gebildete antigene Determinante vom gewünschten Antikörper erkannt und effektiv gebunden wird.

Das für die immunadsorptive Gewinnung der Antikörper verwendete Peptid kann z. B. ein Dekapeptid sein, bevorzugt das Peptid Cys-Arg-Asn-Ala-Ser-Lys-Pro-Gln-Gly-Arg. Erfindungsgemäß werden durch Immunisieren mit synthetischen Peptiden im tierischen System Antikörper induziert und durch Immunadsorption gereinigt. Diese Antikörper reagieren spezifisch mit den zur Immunisierung und Reinigung verwendeten Peptiden. In Abhängigkeit von der Sequenz des verwendeten Peptids binden diese Antikörper entweder nur an Faktor VIIa oder auch, wenn eine im nativen Faktor VII-Molekül exponierte Peptidsequenz gewählt wird, an das intakte Faktor VII-Molekül.

Durch Auswahl entsprechender Peptide als Immunsorbentien können Antikörper selektioniert werden, die spezifisch mit den antigenen Determinanten des Faktors VIIa, die der Erkennungssequenz der Faktor Xa-Spaltstelle dieses Moleküls entsprechen, reagieren. In dem bevorzugten Fall, daß Peptide, die C- bzw. N-terminal die Faktor Xa-Erkennungssequenz aufweisen, sowohl zur Immunisierung als auch zur immunadsorptiven Reinigung verwendet werden, werden Antikörper gegen diese Sequenzen angereichert. Diese reagieren jedoch nicht mit intaktem, nativen Faktor VII, da im intakten Faktor VII-Molekül die Faktor Xa-Spaltstelle entweder nicht stark genug exponiert ist oder nicht die zur antigenen Erkennung erforderliche höhere Struktur aufweist.

Nach den, dem Fachmann an sich bekannten Verfahren können auch monoklonale Antikörper mit den erfindungsgemäßen Eigenschaften hergestellt werden.

Die erfindungsgemäß gewonnenen Antikörper können für, dem Fachmann an sich bekannte, homogene und heterogene Immunoassays wie z. B. Enzymimmunoassays oder freie oder Latex-verstärkte Agglutinationsreaktionen eingesetzt werden. Bevorzugterweise werden sie dazu an einen festen Träger gekoppelt. Solche festen Träger sind dem Fachmann an sich bekannt, wie z. B. Mikrotitrationsplatten, Röhrchen, Kugeln, Microbeads, magnetisierbare Partikel u. ä.. Bevorzugt ist dabei die Immobilisierung an Polystyrolröhrchen oder Mikrotitrationsplatten. Die für die folgenden Immunoassays vorbereiteten Röhrchen können anschließend luftdicht verschlossen z. B. bei 4 °C gelagert werden.

Die erfindungsgemäße Bestimmung des Gehaltes an Faktor VIIa erfolgt durch Vorinkubation der Probe mit solcher Art immobilisierten Antikörpern, wobei die Konzentration des durch die immobilisierten Antikörper gebundenen Faktors VIIa durch eine nachfolgende Inkubation mit einem zweiten Antikörper detektiert wird. Dieser zweite Antikörper muß eine Eigenschaft aufweisen, die meßbar ist, z. B. die Fähigkeit zur Umsetzung oder Bindung eines chromogenen Substrates.

Der zweite Antikörper kann z. B. mit einem Enzym, einem fluoreszierenden Molekül, wie z. B. Fluoresceinisothiocyanat, einer radioaktiven Markierung oder einem zur Chemoluminiszenz fähigen Molekül versehen werden. Bevorzugterweise wird dieser zweite Antikörper mit einem Markerenzym gekoppelt, besonders bevorzugt ist Peroxidase.

Erfindungsgemäß kann mit einem derart immobilisierten Antikörper auch die Konzentration von Faktor VIIa/TF-Komplex bestimmt werden. Voraussetzung ist die Verwendung eines spezifischen Antikörpers gegen Tissue factor als Zweitantikörper, der in beschriebener Weise markiert ist. TF-Antikörper können nach dem Fachmann an sich bekannten Verfahren als polyklonale oder monoklonale Antikörper erhalten werden. Es kann auch der TF-Antikörper gebunden und der Faktor VIIa Antikörper markiert werden.

Die Bestimmung von Faktor VIIa bzw. Faktor VIIa/TF-Komplex kann auch durch gleichzeitige Inkubation der Probe, bevorzugt von Plasma und markiertem Antikörper mit den immobilisierten Antikörpern erfolgen. Darüber hinaus ist ein kompetitives Bestimmungsverfahren möglich, wobei markierter und unmarkierter Faktor VIIa bzw. Faktor VIIa/TF-Komplex um die Bindungsstelle der immobilisierten Antikörper konkurrieren. Der auf diese Weise bestimmte Gehalt an Faktor VIIa läßt eine Aussage über den Aktivierungsgrad des Faktors VII zu. Besonders bevorzugt sind die in den Beispielen angegebenen Ausführungsformen.

Die Beispiele erläutern die Erfindung, schränken sie aber in keiner Weise ein.

In den Beispielen werden folgende Abkürzungen verwendet:
- ELISA: Enzymimmunoassay (enzyme linked immunosorbent assay)
- KLH: Keyhole Limpet hemocyanin (Haemocyanin einer marinen Napfschnecke)
- PBS: Phosphatgepufferte Kochsalzlösung (phosphate buffered saline)
- Tris: Tris (hydroxymethyl)aminomethan
- OD: Extinktion (optical density)
- Cys, C: Cystein
- Ala, A: Alanin Aminosäuren können in D- oder
- Arg, R: Arginin L-Form vorliegen, sofern jedoch
- Pro, P: Prolin nicht extra vermerkt, liegen sie in
- Phe, F: Phenylalanin der L-Form vor.
- Lys,: K Lysin
- Ile, I: Isoleucin
- Gly, G: Glycin
- Glu, E: Glutaminsäure
- Thr, T: Threonin
- Gln, Q: Glutamin
- Boc: t-Butoxycarbonyl
- Fmoc: 9-Fluorenylmethoxycarbonyl
- Mtr: 4-Methoxy-2, 3, 6-trimethylphenylsulfonyl
- DMF: Dimethylformamid
- HoBt: Hydroxybenzotriazol
- DTT: Dithiothreitol
- Trt: Trityl
- Pmc: 2, 2, 5, 7, 8-Pentamethylchroman-6-sulfonyl

### Beispiel 1

### Herstellung eines Antigens zum Immunisieren

a) Synthese von Cys-Arg-Asn-Ala-Ser-Lys-Pro-Gln-Gly-Arg 1 g Fmoc-Arg (Pmc)-p-alkoxybenzylesterharz wurde 2 x mit 15 ml DMF 1 min gewaschen und die Fmoc-Gruppe mit 15 ml 20 % Piperidin/ DMF (V/V) (1 x 3 min, 1 x 10 min) abgespalten. Das Harz wurde daraufhin je 3 x mit DMF bzw. Isopropanol (je 15 ml) und 2 x mit 15 ml DMF gewaschen. Es wurden 1.5 mmol Fmoc-Aminosäure und 2.25 mmol HOBt in 15 ml DMF gelöst zum Harz gegeben und nach Zusatz von 1.65 ml einer 1 M Diisopropylcarbodiimid-lösung in Dichlormethan 1.5 h bei Raumtemperatur geschüttelt. Die Reaktion wurde mit einem Ninhydrintest auf Vollständigkeit geprüft. Danach wurde das Harz je 3 x mit DMF bzw. Isopropanol (je 15 ml) gewaschen und ein neuer Zyklus begonnen. Als letzte Aminosäure wurde eine Boc-Cys (Trt) benutzt. Das Harz wurde 3 x mit je 15 ml Isopropanol und Diethylether gewaschen und im Hochvakuum getrocknet. 1.9 g Harz wurden mit 1 ml Thioanisol, 1 ml Ethandithiol und 18 ml Trifluoressigsäure 2 h bei Raumtemperatur gerührt, abfiltriert, das Harz mit 3 Portionen Trifluoressigsäure/Dichlormethan (1:1) gewaschen und die Filtrate in Ether kristallisiert. Das Rohpeptid wurde mit Diethylether gewaschen und getrocknet. Das Peptid wurde an ^{R}Sephadex G 25 in 0.5 % Essigsäure chromatographiert. Ausbeute: 649 mg.
   100 mg dieses Produktes wurden zur Weiterreinigung an einer präparativen HPLC-Anlage an reversed-phase Material chromatographiert (0.1 % Acetonitril, Gradientenbetrieb). Der Peptidpool wurde gefriergetrocknet. Ausbeute: 48 mg.
b) Konjugatherstellung
   20 mg KLH wurden in 0.05 mM Natriumphosphatpuffer pH 8.0 gelöst und mit 2 mg gamma-Maleimidobuttersäurehydroxysuccimidester 1 h lang gerührt. Das Protein wurde an einer ^{R}Sephadex G 50-Säule (2 x 30 cm) (0.1 M Natriumphosphat, 0.5 mM EDTA, pH 6.0) chromatographiert. Das Eluat wurde auf 5 ml konzentriert und mit 20 mg Peptid 1 h inkubiert. Nach Dialyse und Lyophilisation wurden 28 mg Peptidkonjugat erhalten.

### Beispiel 2

### Immunisieren von Kaninchen

5 Kaninchen wurden mit jeweils 2 mg Antigen pro Tier über einen Zeitraum von 8 Wochen immunisiert; die Applikationen des Peptid-KLH-Konjugats erfolgten subcutan und intravenös. Danach wurden die Tiere entblutet, die gewonnenen Rohantiseren gepoolt und mit Konservierungsmittel stabilisiert. Ausbeute: 175 ml Antiserum pro Tier.

### Beispiel 3

### Herstellen von Immunadsorbentien

Für die affinitätschromatographische Reinigung der Rohantiseren wurden ca. 30 mg Dekapeptid der Sequenz Cys-Arg-Asn-Ala-Ser-Lys-Pro-Gln-Gly-Arg (wie in Beispiel 1 a hergestellt) an einer Festphase kovalent immobilisiert. Die Kopplungsreaktion erfolgte mit Bromcyan-aktivierter Sepharose nach einem beschriebenen Verfahren (Axen, R. et al., Nature, 214, 1302, 1967). Anschließend wurde das Immunadsorbens jeweils mit phosphatgepufferter Kochsalzlösung (PBS; 0,15 mol/l, pH 7.2) und Essigsäure 0,5 ml/l, pH 2.5) gewaschen. Vor der Verwendung wurde das Adsorbens mit dem 3fachen Gelvolumen PBS äquilibriert. Ausbeute: ca. 30 ml Peptid-Sepharose.

### Beispiel 4

### Isolieren spezifischer Antikörper

100 ml Rohantiserum wurden auf die mit PBS äquilibrierte 30 ml Peptid-Sepharose (1.6 x 15 cm) aufgetragen und anschließend mit PBS gewaschen, bis die Extinktion bei 280 nm 0.01 betrug. Danach erfolgten Waschschritte mit Kochsalzlösung (1 mol/l, pH 7.0) und Wasser (pH 7.0), wobei jeweils das 3fache Gelvolumen verwendet wurde. Die Elution der Antikörper vom Immunadsorbens erfolgte mit Essigsäure (0.1 mol/l, pH 2.5), die Antikörperlösung wurde mit festem Natriumphosphat (0.01 mol/l) auf pH 7.0 eingestellt, konzentriert (Amicon-Membran) und bei -70 °C gelagert. Ausbeute: ca. 35 mg Antikörper.

### Beispiel 5

### Testen der immunadsorptiv gewonnenen Antikörper

a) Herstellen von Antikörper-beschichteten Röhrchen
   Die in Beispiel 4 gewonnenen Antikörper wurden mit Tris-Pufferlösung (0,025 mol/, pH 7.6) auf eine Konzentration von 5 µg/ml verdünnt und durch Adsorption an Polystyrolröhrchen immobilisiert. Pro Röhrchen wurden 250 µl Antikörperlösung für 20 Stunden bei 20 °C inkubiert, anschließend die Flüssigkeit abgesaugt und die Röhrchen luftdicht verschlossen bei 4 °C gelagert.
b) Durchführung des Enzymimmunoassays (ELISA)
   Die zu testenden Proben wurden mit Inkubationspuffer (50 mM Tris, 100 mM NaCl, 0,1 % Azid, pH 7.2) 1+1 verdünnt und jeweils 200 µl pro Röhrchen (siehe Beispiel 5a) 30 min bei 37 °C inkubiert. Anschließend wurde die Inkubationslösung entfernt und das Röhrchen zweimal mit je 500 µl Waschlösung (0.02 mol/l Natriumphosphat, 0,05 % Tween, pH 7.6) gewaschen. Anschließend wurden 200 µl Peroxidase-konjugierter Anti-Tissue Factor-Antikörper zugefügt und die Röhrchen 30 min bei 37 °C inkubiert. Nach Entfernen der Konjugat-Lösung und zweimaligem Waschen wurden 200 µl Substrat-Chromogen-Lösung (Wasserstoffperoxid; o-Phenylendiamin) zugefügt und die Röhrchen bei Raumtemperatur inkubiert. Nach ½stündiger Inkubation wurde die Peroxidase mit Schwefelsäure inaktiviert und die Extinktion der Reaktionslösung bei 492 nm bestimmt.
c) Bestimmung von in vitro-gebildetem Faktor VIIa-TF-Komplex mittels Enzymimmunoassay
   In einem in vitro-Versuch wurde Plasma mit einer Thromboplastin-Lösung versetzt und 150 min bei 37 °C inkubiert. Danach wurde die Probe 1+9 mit PBS verdünnt und die gebildete Faktor VIIa-TF-Konzentration im ELISA bestimmt. In der nachfolgenden Tabelle sind die Extinktionswerte (492 nm) einer Plasmaprobe zum Zeitpunkt der Zugabe von Thromboplastin sowie 150 min nach der Zugabe dargestellt; als Vergleich dient die Extinktion eines Röhrchens ohne Plasma.

**Tabelle 1**

| Probe | OD₄₉₂/30 min |
|---|---|
| Plasma + Thromboplastin (0 min Inkubation) | 0.34 |
| Plasma + Thromboplastin (150 min Inkubation) | 0.82 |
| Puffer-Leerwert | 0.16 |

In einem weiteren Experiment wurde die Spezifität der Antikörper gegen Faktor VIIa überprüft. Mit Citratlösung antikoaguliertes Plasma wurde mit Thromboplastin-Lösung versetzt und bei 37 °C inkubiert. Zu verschiedenen Zeiten wurden Aliquote entnommen und die Reaktion durch Zugabe von Citrat (Endkonzentration: 0.15 mol/L) abgestoppt. Die Proben wurden mit Inkubationspuffer 1+1 verdünnt und mit dem ELISA getestet.

Die Tabelle gibt die Ergebnisse wieder:

**Tabelle 2**

| Zeit (min) | OD₄₉₂/30 min |
|---|---|
| 0 | 0.25 |
| 5 | 0.39 |
| 10 | 0.53 |
| 20 | 0.60 |
| 60 | 0.71 |
| 75 | 0.79 |
| Plasma-Leerwert | 0.24 |

Die Ergebnisse zeigen, daß der Faktor VIIa-TF-Komplex auf diese Weise quantitativ gemessen werden kann: während der Aktivierungsreaktion steigt die Konzentration von Faktor VIIa-TF-Komplex mit zunehmender Zeit an.

Die erfindungsgemäßen Peptide, die eine Aminosäuresequenz aufweisen, die ganz oder teilweise der Aminosäuresequenz von Faktor VII entspricht und antigen ist, induzieren somit die bindungsspezifischen Antikörper gegen die jeweiligen im Peptid vorhandenen antigenen Determinanten. Diese spezifischen Antikörper können anschließend durch Immunadsorption an Peptiden mit der gleichen antigenen Determinante gereinigt werden. Die Verwendung synthetischer Peptide hat den wesentlichen Vorteil, daß absolute reine Antigene zur Immunisierung eingesetzt werden, so daß im resultierenden Antiserum keinerlei Kreuzreaktion mit anderen Proteinen oder anderen Teilen des Faktor VII-Moleküls auftreten kann. Erfindungsgemäß wird bevorzugt ein Peptid, das der C- bzw. N-terminalen Aminosäurensequenz der Faktor Xa-Spaltstelle im Faktor VII-Molekül entspricht, verwendet. Mit einem Antikörper gegen dieses Peptid lassen sich nur gespaltene Faktor VII-Moleküle nachweisen, d.h. Faktor VIIa, da im intakten nativen Faktor VII diese Sequenz der Antikörpererkennung nicht zugänglich ist. Die Messung der Menge gebundenen Antikörpers mittels ELISA erlaubt einen direkten Rückschluß auf die Konzentration von gebildetem Faktor VIIa bzw. Faktor VIIa-TF-Komplex und damit eine Aussage über den Aktivierungsgrad von Faktor VII.

## Patentansprüche

1. Diagnostisches in vitro Verfahren zur Bestimmung von F VIIa bzw. F VIIa/TF-Komplex, dadurch gekennzeichnet, daß Antikörper eingesetzt werden, die durch Immunisierung mit Peptiden aus F VIIa erhältlich sind, wobei diese Peptide mindestens die 4 carboxy-terminalen Aminosäuren - Pro-Gln-Gly-Arg-OH und/oder aminoterminalen H-Ile-Val-Gly-Gly endständig enthalten.

2. Diagnostisches Verfahren nach Anspruch 1, dadurch gekennzeichnet, daß ein Antikörper an eine Festphase gebunden ist und der zweite Antikörper eine nachweisbare Funktion trägt.

3. Diagnostisches Verfahren nach Anspruch 1 oder 2, dadurch gekennzeichnet, daß ein erster Antikörper, erhältlich durch Immunisierung mit Peptiden aus
F VIIa, wobei diese Peptide mindestens die 4 carboxy-terminalen Aminosäuren -Pro-Gln-Gly-Arg-OH und/oder aminoterminalen H-Ile-Val-Gly-Gly endständig enthalten, an die Festphase gebunden ist und der zweite Antikörper, der eine Nachweisfunktion trägt, gegen TF gerichtet ist.

4. Diagnostisches Verfahren nach Anspruch 1, dadurch gekennzeichnet, daß zur Immunisierung Dekapeptide eingesetzt werden, die gegebenenfalls ein N- oder C-terminal eingefügtes Cystein enthalten.

5. Dekapeptide Cys-Arg-Asn-Ala-Ser-Lys-Pro-Gln-Gly-Arg und Ile-Val-Gly-Gly-Lys-Val-Cys-Pro-Lys-Gly.

6. Dekapeptide nach Anspruch 5, dadurch gekennzeichnet, daß sie zur besseren Immunisierung an ein Trägermolekül gekoppelt sind.

7. Konjugate aus synthetischen Peptide aus F VIIa, die mindestens die 4 carboxyterminalen Aminosäuren (-Pro-Gln-Gly-Arg-OH) und/oder aminoterminalen (H-Ile-Val-Gly-Gly-) Aminosäuren endständig enthalten, wobei gegebenenfalls ein Cystein auf dem den 4 genannten terminalen Aminosäuren entgegengesetzten Ende angefügt ist, ausgenommen das Undekapeptid Glu-Lys-Arg-Asn-Ala-Ser-Lys-Pro-Gly-Pro-Arg und die jeweilige vollständige Kette von F VIIa und einem Trägermolekül.

## Claims

1. An in vitro diagnostic method for determining F VIIa or F VIIa/TK complex, wherein antibodies obtainable by immunization with peptides from F VIIa are employed, these peptides comprising terminally at least the 4 carboxy-terminal amino acids -Pro-Gln-Gly-Arg-OH and/or amino-terminal H-Ile-Val-Gly-Gly.

2. The diagnostic method as claimed in claim 1, wherein one antibody is bound to a solid phase and the second antibody carries a detectable functionality.

3. The diagnostic method as claimed in claim 1 or 2, wherein a first antibody obtainable by immunization with peptides from F VIIa, these peptides comprising terminally at least the 4 carboxy-terminal amino acids -Pro-Gln-Gly-Arg-OH and/or amino-terminal H-Ile-Val-Gly-Gly, is bound to the solid phase, and the second antibody which carries a detectable functionality is directed against TF.

4. The diagnostic method as claimed in claim 1, wherein decapeptides which optionally comprise an N- or C-terminally inserted cysteine are employed for the immunization.

5. A decapeptide Cys-Arg-Asn-Ala-Ser-Lys-Pro-Gln-Gly-Arg or Ile-Val-Gly-Gly-Lys-Val-Cys-Pro-Lys-Gly.

6. A decapeptide as claimed in claim 5, which is coupled to a carrier molecule for better immunization.

7. Conjugates of synthetic peptides from F VIIa which comprise terminally at least the 4 carboxy-terminal amino acids(-Pro-Gln-Gly-Arg-OH)and/or amino-terminal (H-Ile-Val-Gly-Gly-) amino acids, a cysteine optionally being attached at the opposite end to the 4 terminal amino acids mentioned, excepting the undecapeptide Glu-Lys-Arg-Asn-Ala-Ser-Lys-Pro-Gly-Pro-Arg and the respective complete chain of F VIIa, and of a carrier molecule.

## Revendications

1. Procédé de diagnostic in vitro pour la détermination du facteur VIIa (F VIIa) ou du complexe F VIIa/TF, caractérisé en ce que l'on utilise des anticorps qui peuvent être obtenus par immunisation avec des peptides de F VIIa, ces peptides comportant en bout de chaîne au moins les 4 aminoacides carboxy-terminaux Pro-Gln-Gly-Arg-OH et/ou les 4 aminoacides amino-terminaux H-Ile-Val-Gly-Gly.

2. Procédé de diagnostic selon la revendication 1, caractérisé en ce qu'un anticorps est fixé à une phase solide et le second anticorps porte une fonction détectable.

3. Procédé de diagnostic selon la revendication 1 ou 2, caractérisé en ce qu'un premier anticorps, pouvant être obtenu par immunisation avec des peptides de F VIIa, ces peptides comportant en bout de chaîne au moins les 4 aminoacides carboxy-terminaux Pro-Gln-Gly-Arg-OH et/ou les 4 aminoacides amino-terminaux H-Ile-Val-Gly-Gly, est fixé à la phase solide, et le second anticorps, qui porte une fonction détectable, est dirigé contre TF.

4. Procédé de diagnostic selon la revendication 1, caractérisé en ce que l'on utilise pour l'immunisation des décapeptides qui comportent éventuellement un résidu cystéine introduit à l'extrémité N- ou C-terminale.

5. Décapeptides Cys-Arg-Asn-Ala-Ser-Lys-Pro-Gln-Gly-Arg et Ile-Val-Gly-Gly-Lys-Val-Cys-Pro-Lys-Gly.

6. Décapeptides selon la revendication 5, caractérisés en ce que, pour une meilleure immunisation, ils sont couplés à une molécule porteuse.

7. Conjugués de peptides synthétiques de F VIIa qui comportent en bout de chaîne au moins les 4 aminoacides carboxy-terminaux Pro-Gln-Gly-Arg-OH et/ou les 4 aminoacides amino-terminaux H-Ile-Val-Gly-Gly, un résidu cystéine étant éventuellement introduit à l'extrémité opposée aux 4 aminoacides terminaux cités, à l'exception de l'undécapeptide Glu-Lys-Arg-Asn-Ala-Ser-Lys-Pro-Gly-Pro-Arg, et la chaîne complète respective de F VIIa et une molécule porteuse.
